# EUROPEAN PATENT APPLICATION

(11) **EP 4 477 107 A1**
(43) Date of publication of application: **18.12.2024**
(21) Application number: 23178612.0
(22) Date of filing: 12.06.2023
(51) Int. Cl.: A46B 15/00, A61B 5/00, A61B 5/11, A61B 5/22, A61F 7/00

(54) **A HAND-HELD PERSONAL CARE DEVICE**

(71) Applicant: Koninklijke Philips N.V., 5656 AG Eindhoven (NL)
(72) Inventor: HIWALE, Sujitkumar, Eindhoven (NL); DE RUYTER, Boris Emmanuel Rachmund, Eindhoven (NL); JOHNSON, Mark Thomas, 5656AG Eindhoven (NL); KOOIJMAN, Gerben, Eindhoven (NL); GERHARDT, Lutz Christian, Eindhoven (NL); VAN DEN DUNGEN, Wilhelmus Andreas Marinus Arnoldus Maria, Eindhoven (NL)
(74) Representative: Philips Intellectual Property & Standards

(57) **Abstract**

A hand-held personal care device has an actuator, for implementing a personal care function, which generates a vibration (or multiple different vibrations). A sensor arrangement detects a signal which varies in dependence on the vibration of the personal care device. By analyzing the signal, a user's grip pattern on the handle is detected. This can be used to detect problems the user is having in creating a grip caused by a physical disability such as joint stiffness caused by arthritis.

## Description

### FIELD OF THE INVENTION

This invention relates to personal care devices, and in particular it relates to the detection of physical disability affecting the grip of a user, by detecting their grip when using the personal care device.

### BACKGROUND OF THE INVENTION

Morning stiffness (lasting from a few minutes to a few hours) is a common and clinically important symptom in patients with arthritis and peripheral vascular diseases (PVD). Morning stiffness often affects small joints, like those in the fingers, wrists, and toes. Morning stiffness prevalence has been reported in the range of 0.4%-1.3% at the overall population level (general U.S. population); however, it increases significantly in the elderly population.

Various other conditions are also known to affect motor coordination. These include osteoarthritis (with a prevalence of 50% in the population over 65 years of age), gout, rheumatoid arthritis, peripheral artery diseases, and other diseases and injuries, such as strains and sprains, carpel tunnel syndrome (CPT), sports-related injuries or occupational injuries etc.

Joint stiffness can mean that a person is disabled due to joint stiffness and pain for a quite significant time early in the morning. This is likely to hamper many basic self-care activities in morning, which require a good amount of motor coordination such as brushing, shaving, bathing etc. These activities are often referred as Activities of Daily Living (ADLs). This can lead to major changes in daily routine, absenteeism from work or even psychological issues.

In the context of oral health, poor motor coordination can lead to poor brushing quality, which can lead to suboptimal oral hygiene, which ultimately can lead to oral diseases. Therefore, it would be important in such cases to provide some means to diagnose a physical disability passively without the need for any additional devices. This information can be then used to provide some mechanism to compensate joint stiffness so that a person can perform the required physical activity with minimum discomfort.

The most common method for diagnosis of morning stiffness or physical disability is analysis of a patient's history and thorough physical examination. However, this requires a visit to a physician's office, which may not be convenient, especially in the elderly population. Given a slow progression of disease, the disability could be missed during routine examination or could be quite significant by the time it is diagnosed by a physician. Another disadvantage is the subjective nature of examination, which makes it difficult to assess disease progression or to judge improvement after treatment.

An objective method of diagnosis of physical disability involves assessment of physical activity (PA) and its categorization. PA during ADLs can be categorized into different intensity levels (e.g., low intensity, medium intensity, or high intensity) based on the energy used for a given task i.e., Physical Activity Energy Expenditure (PAEE). The doubly labelled water technique is considered as a gold standard for measuring PAEE. However, given its limitation for practical use, various other techniques, or surrogate measures (most common and simplest being use of questionnaires) are used to quantify PA in routine practice.

An accelerometer-based technique has been also proposed as a proxy to measure and quantity PA. Accelerometers measure PA by quantifying movement and this has shown to be highly proportional to PAEE measured by the doubly labelled water technique. It has been reported in literature that accelerometer signals obtained near a wrist joint can be used to assess physical disability for performing activities of daily living (ADL), such as brushing, with a fair accuracy. For daily use, a disadvantage of accelerometer-based methods comes from an obligation on user to wear such a senor (e.g., a wrist band) all the time without fail; it is also associated with additional cost and may not be useful in elderly people due to the known issue of poor compliance (with any additional device) in this age group.

As explained above, arthritis and other similar physical disabilities may develop rather slowly and it is difficult to detect them at early stages using conventional methods. There is a subjective element to physical assessment and there is no solution which fits well into a daily workflow. Disease progression trends are also difficult to obtain using conventional methods, which makes it difficult to assess disease progression or improvement after treatment.

There is therefore a need to detect physical conditions, which affect a user's ability to grip, at an early stage. This would then allow for earlier or targeted interventions and can result in improved compliance to personal care or health care routines.

### SUMMARY OF THE INVENTION

The invention is defined by the claims.

According to examples in accordance with an aspect of the invention, there is provided a hand-held personal care device comprising:
a handle;
an actuator for implementing a personal care function which generates a vibration;
a sensor arrangement for detecting a signal which varies in dependence on the vibration of the personal care device;
a processing arrangement, configured to:
   detect a user's grip pattern on the handle, in use of the personal care device, by sensing, using the sensor arrangement, a modulation of the vibrations generated by the actuator depending on the user's grip pattern.

The invention enables detection of the grip pattern of a user when holding a personal care device, by monitoring the identifiable effect of their particular grip pattern on the vibration of the device. The device vibrates in use as part of the delivery of the personal care function, and the way the user grips (i.e., holds) the device functions as a damping or tuning to those vibrations, for example at one or more frequencies. This damping influences the resulting vibration characteristics, so that sensing of the vibration of the device (directly or indirectly) enables the type of grip to be determined.

By "modulation" of the vibrations is meant any alteration to the vibratory response of the device to a given actuator command. This modulation a may be a linear or non-linear frequency response, and may be at fundamental and/or harmonic frequencies.

Knowledge of the type of grip can be used to detect difficulties the user is having in adopting a normal grip, because of finger and/or wrist stiffness. Thus, it can be used to detect stiffness issues at an early stage.

The processing arrangement is for example configured to classify the user's grip pattern as a palm grip or a finger grip or a combined palm and finger grip. Different grips and grip sequences may be indicative of different types of disability.

The processing arrangement is for example configured to classify the finger grip as a stiff finger grip or a claw finger grip. Different finger grips may also indicate different types of stiffness and hence underlying issues.

In a first set of examples, the processing arrangement comprises a band pass filter arrangement, for example with multiple frequencies. This is one way to analyze the sensor signal to extract information which varies in dependence on the grip pattern.

In a second set of examples, the processing arrangement comprises a neural network. The neural network is trained to detect different grip patterns from the vibration signal.

The neural network is for example supplied with a spectrogram of the sensor signal for a continuous range of frequencies up to at least double the frequency of the vibrations generated by the actuator. This range may be up to a maximum frequency above 100Hz, or 500Hz or 1kHz. Thus, the frequency characteristics for a broad spectrum may be analyzed by the neural network.

In all examples, the processing arrangement may be further configured to:
monitor changes in grip pattern over time; and
assess a presence and/or progression of a physical limitation of the user in generating a hand grip based on the grip pattern changes over time.

Over a short time period, changes in grip pattern, and using specific grips and grip sequences, may indicate difficulty in maintaining a particular grip, and be indicative of particular disabilities. Over a longer time period, the progression of a disability may become evident.

The processing arrangement is for example further configured to determine if the grip pattern changes from a first grip to a second grip with a grip pause between. This pause may indicate issues the user is having with reorienting the device.

The processing arrangement is for example configured to determine if the grip pattern changes from a first palm grip to a second palm grip with a grip pause between, and to provide an indication of finger stiffness in response.

This may indicate that a user cannot perform a finger grip (which would give more control over the orientation of the device), because when they adjust their grip, it is repeatedly to palm grips.

Another indicator may be a change in orientation by using two hands or when not using the personal care device.

The processing arrangement may be configured to determine if the device orientation changes between the first and second palm grips and provide an indication of wrist stiffness in response. Repositioning the device between palm grips is an indicator of wrist stiffness.

When monitoring grip pattern changes over time, the processing arrangement may be configured to provide:
an indication of finger stiffness in response to a change from a stiff finger grip to a palm grip; or
an indication of finger stiffness in response to a change from a stiff finger grip to another stiff finger grip; or
an indication of an absence of joint stiffness in response to a change from a stiff finger grip to a claw finger grip; or
an indication of wrist stiffness in response to repositioning of the device during release between successive stiff finger grips.

In one example, the sensor arrangement comprises an accelerometer. This may be an existing sensor of the personal care device, for directly measuring movement of the device. However, a simple single axis accelerometer (e.g., a microphone may be considered to be a single axis accelerometer) is sufficient to detect vibrations, and the motion direction is not needed.

In another example, the sensor arrangement comprises a current sensor for sensing the actuator (e.g., motor) drive signal. In this case, the damping of the vibrations and detuning of the non-linear system result in a different load to the actuator and the change in load modulates the actuator current. Thus, the actuator current may be interpreted to determine the type of grip.

The device may further comprise a therapy delivery system for delivering thermal therapy to the user. This may be used to relieve discomfort and/or provide treatment for the condition.

The therapy delivery system for example comprises a heating or cooling system for delivering heating or cooling targeted to the finger joints. If the grip is known, it is possible to target the heating or cooling to the finger joint areas for that grip.

In one set of examples, the device comprises a powered toothbrush.

The invention also provides a method of detecting a user's grip pattern, comprising:
receiving a sensor signal which varies in dependence on a vibration of a hand-held personal care device which is actuated to implement a personal care function which involves driven vibrations; and
processing the sensor signal to detect a modulation of the vibrations generated by the actuator depending on the user's grip pattern and thereby determine the grip pattern.

The invention also provides a computer program comprising computer program code means which is adapted, when said program is run on a processor, to perform the method defined above.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 shows an example of an electric toothbrush able to detect a user's grip pattern;
Fig. 2 shows a FFT plot of FFT amplitude (y-axis) versus frequency, for a frequency range 0 to 8000Hz;
Fig. 3 shows a first example of a processing arrangement, to detect grip types from the sensor signal;
Fig. 4 shows a second example of a processing arrangement, to detect grip types from the sensor signal;
Fig. 5 shows how the grip pattern is used to determine if further investigation is needed;
Fig. 6 shows an example of monitoring after an initial palm grip is detected; and
Fig. 7 shows an example of monitoring after a stiff finger grip is detected.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a hand-held personal care device having an actuator, for implementing a personal care function, which generates a vibration (or multiple different vibrations). A sensor arrangement detects a signal which varies in dependence on the vibration of the personal care device. By analyzing the signal, a user's grip pattern on the handle is detected. This can be used to detect problems the user is having in creating a grip caused by a physical disability such as joint stiffness caused by arthritis.

Joint stiffness in the fingers or wrist is likely to affect how a person holds a personal care device, such as a powered toothbrush. Therefore, by detecting the hand grip pattern it is possible to get information about joint stiffness in the fingers or wrist.

The invention may be applied to any personal care device which generates a vibration when performing the personal care function. The personal care function may be a function for promoting health or for treating a medical condition or it may be for a personal hygiene routine.

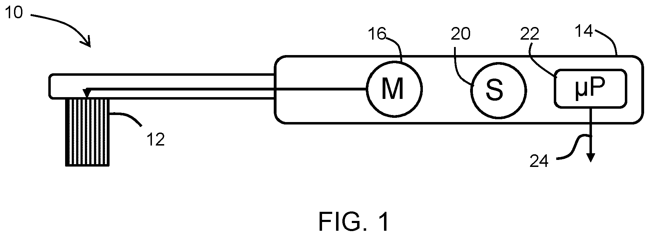

Fig. 1 shows an example of an electric toothbrush 10 having a driven head 12 and a handle 14, with an actuator 16 in the handle for driving the head. The actuator in one example is a motor and the head is driven to rotate, and this generates a vibration which is transmitted to the device body. A sensor 20 generates a sensor signal which depends on motion and vibrations of the toothbrush.

A processing arrangement, schematically shown as processor 22, detects a grip pattern from the sensor signal and thereby detects a difficulty the user is facing creating a correct grip. An output 24 is generated which indicates the detected grip pattern and/or an analysis of the grip patterns over time.

One option is that the sensor comprises a motor current sensor. In this case, the grip pattern mechanically modulates or dampens the driven vibrations of the device, and the grip pattern thereby acts as a specific load pattern to the motor. The change in load modulates the motor current. Thus, the motor current may be interpreted to determine the presence and type of grip being applied by the user. Another example is an accelerometer, such as a single axis accelerometer, which in its simplest form may be a microphone. A direction vector for the vibrations is not needed. Only the frequency and amplitude characteristics of the vibration are needed to detect the modulation created by the grip of the user. In the case of a microphone, the sounds picked up by the microphone, or more generally vibrations picked up by an accelerometer, will not be limited to sounds transmitted by vibration of the device, but will include any sounds made by the device even if for example the vibrations are isolated from the housing of the device to which the microphone is mounted.

A user can hold a personal care device in different ways by using different combinations of the fingers and palm.

A palm grip involves wrapping the fingers all around the handle to form a closed fist around the handle. A finger grip involves holding the handle by the fingers with less or no support from the palm. A loose palm grip may be considered to combine the palm grip and finger grip characteristics in that both the pam and fingers are involved in holding the device. A finger grip may be a stiff finger grip where the handle is clamped between the thumb and bases of the fingers (rather than the tips) and the fingers remain outstretched, whereas a claw finger grip makes use only of the tips of the thumb and fingers with the fingers curled.

The vibration response will change depending on the grip used by a user to hold the device. Experiments have shown that different grips can be reliably identified based on the vibration response patterns.

By way of example, a sample of the sensor signal over a time period, such as 1 second, may be converted to a spectrogram. The signal is for example sampled at 1600Hz. The frequency response for different grip patterns is readily detectable from the spectrogram image.

To process the sensor signal, a first approach is based on basic signal processing in the frequency domain.

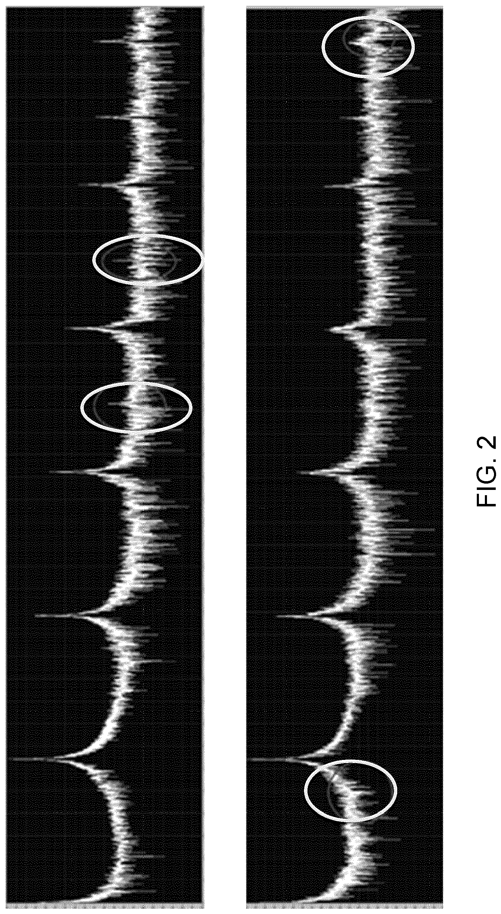

Fig. 2 shows a FFT plot of FFT amplitude (y-axis) versus frequency, for a frequency range 0 to 8000Hz.

The top plot is for a toothbrush held with a finger grip and the bottom plot is for a toothbrush held with a full (palm + finger) grip.

Significant differences between the plots arise at various characteristic frequency bands. A system of narrow band pass filters can thus be used to classify different grips. For example, based on Fig. 2, the use of narrow band filters in the area of 4420Hz and 5780Hz may be used to distinguish between the two grip patterns. These areas are shown with rings. The output of an array of narrow band pass filters tuned to specific frequency bands can thus be combined and a simple threshold may be used to classify different grips. More advanced filters that also include the power over a wider range can be added, such as areas around 1kHz and 7660Hz in this particular example, also shown with rings.

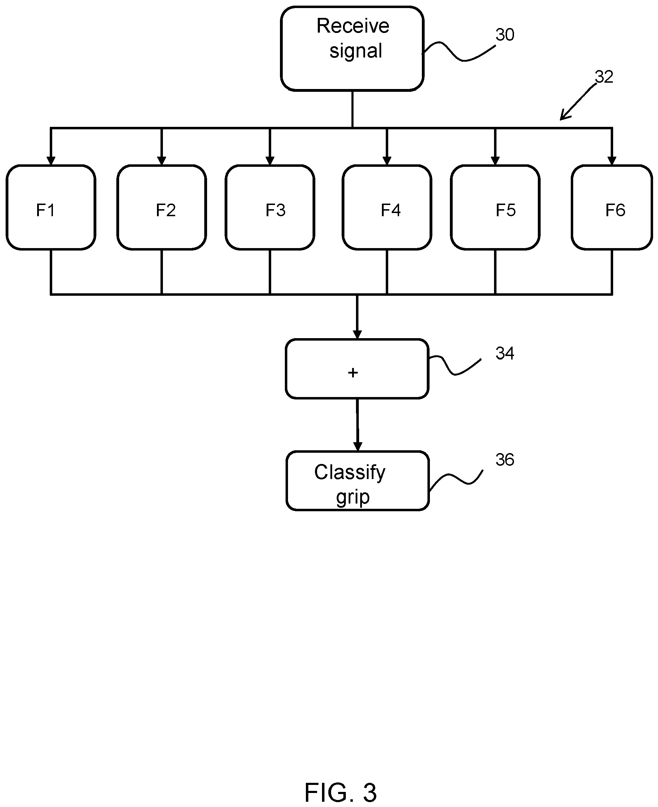

Fig. 3 shows a first example of a processing arrangement, to detect grip types from the sensor signal, by filtering out characteristic frequency bands from the vibration of the toothbrush.

In step 30, the vibration signal is received. In step 32 a set of narrowband filters is used, each designed for a frequency range F 1 to F6 which is found to be characteristic of particular type of grip.

The outputs of the filters are combined in step 34, and a threshold comparison in step 36 is used to classify the grip types (or no grip).

A second approach for the processing of the sensor signal uses a trained Artificial Intelligence (AI) based model.

To demonstrate the feasibility, tests have been performed for classifying different grips with normal and stiffened finger and wrist joints while brushing. The same approach can be also used to detect changes in grips or pauses between the grips when the forces on the handle are substantially released while it is being repositioned.

During this repositioning, the system is only slightly damped or loaded so the vibrations are much more free or floating.

The AI model is for example a classic CNN classifier with a limited number of layers. For example, algorithms of 3 to 5 layers have all be found to perform well. These small CNN-based algorithms can easily be used in embedded firmware in for example the toothbrush handle. Of course any suitable AI approach may be employed such as a fully connected network approach.

The input for training the AI algorithm is a timeseries of the sensor signal obtained from the toothbrush while holding it in different grip positions. The AI may be trained for an accelerometer signal, motor current signal or microphone signal or any other sensor which may function as a vibration sensor. The input from the sensor is first converted to a spectrogram image.

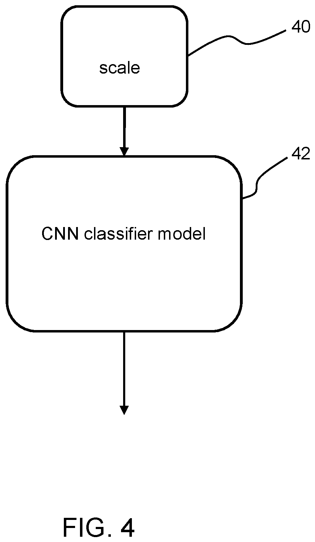

Fig. 4 shows a second example of a processing arrangement, to detect grip types from the sensor signal, based on the AI approach. A spectrogram image is formed of a time window of vibration, such as a 1 second vibration signal sample, converted into an image. The image is then fed into a simple CNN based classifier model.

In step 40, the spectrogram image is scaled, and it is fed into the CNN model 42, comprising an input layer and convolution layers. The AI model then generates a grip classification. Even after use of a limited datasets for training, it has been found that the algorithms can successfully classify the correct grip pattern in almost all cases.

A simplest implementation uses a single sensor e.g. accelerometer for the vibration sensing. However, it is also possible to use data from other embedded sensor in the device to increase specificity of the detection.

Different grip types and different changes in grip type may trigger different required actions and investigations.

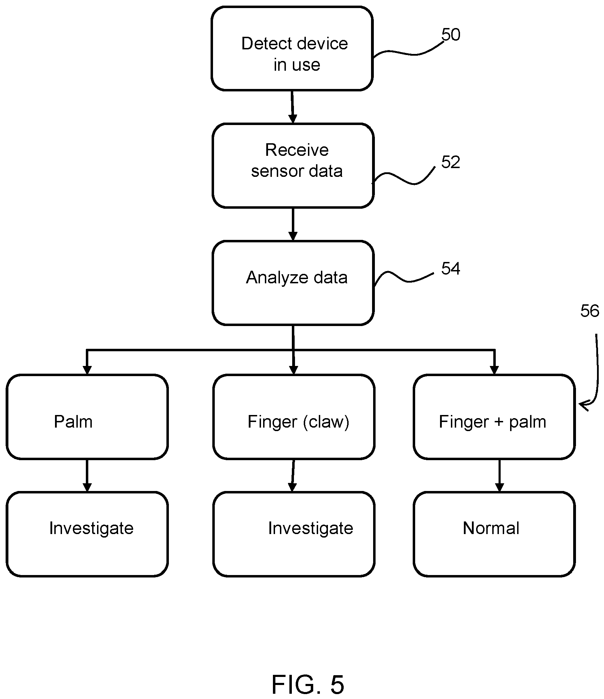

Fig. 5 shows how the grip pattern is used to determine if further investigation is needed.

In step 50, operation of the device is detected. In step 52, the sensor data is received, and it is analyzed in step 54. The grip can be categorized into of three different types shown by row 56; palm grip, finger grip or combined palm and finger grip.

In normal circumstances, a user is likely to hold a device using both finger and palm surfaces. This grip provides firm support as well as a flexibility to maneuver the device into different positions. Therefore, such grip indicates a normal response and less possibility of any finger or wrist joint stiffness. The other two grips, such as predominant use of the palm surface (palm grip) or fingers (finger grip) indicate a possibility of some limitation in holding the device and warrant a further investigation.

The grip pattern information can be used for further classification of grips and to identify the underlying pathology. In particular, the changes in grip pattern over time, and periods of released grip (e.g., where the device is repositioned), and how rotation of the device changes during this regripping, provide further insights.

When a palm grip is detected, the grip pattern may then be continuously monitored to detect any change in grip during use of the device. During device use, a user can change the grip pattern or sometimes may pause for few second before changing to the next grip.

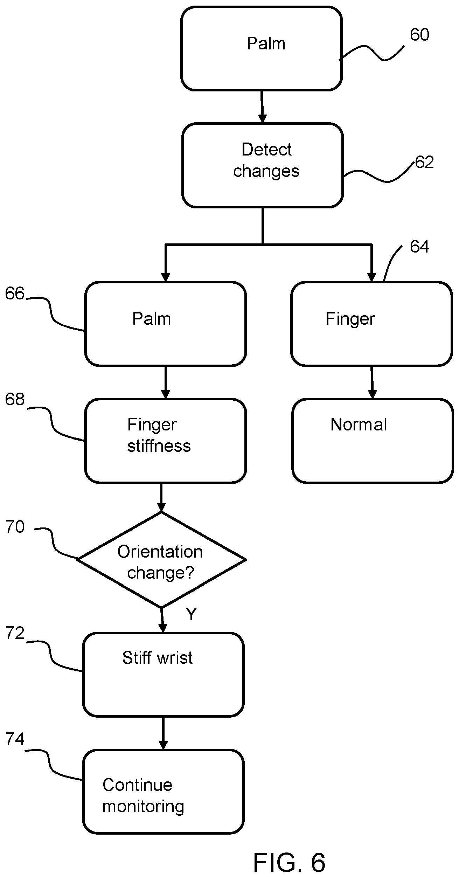

Fig. 6 shows an example of monitoring after an initial palm grip is detected in step 60. Changes in grip pattern are monitored in step 62. If the next grip pattern is a finger grip as detected in step 64, this indicates a normal response. If the next grip is another palm grip as detected in step 66, this indicates finger stiffness as concluded in step 68. In particular, if the grip pattern changes repeatedly to a same palm grip after release times, it indicates that user is not able to use the fingers optimally for controlling the device and it can be assumed that there is a high probability of finger stiffness.

Users with wrist stiffness find it difficult to reach to all the areas on face or mouth. Such users are typically likely to change the orientation of the device during release times, for example rotating the device by 90 degrees to reach to new oral locations while retaining the same grip. The device movement moment can be detected based on the vibration pattern, similarly to the grip detection. In addition to that, a motion sensor such as accelerometer, magnetometer or gyroscope can detect the repositioning or rotation of the device (which may be a gradual or abrupt change in orientation) during this release time (which may be considered to be a free or floating moment). Therefore, abrupt changes in brush orientation as detected in step 70 can be considered as an indication of user having a stiff wrist joint as concluded in step 72. The monitoring continues in step 74.

When the user repeatedly uses the palm grip but without repositioning the device then the wrist joint is likely to have a good reach, and the problem is likely to be more in the finger joints.

The finger grip can be further classified into two different categories; claw finger grip (normal bending at interphalangeal joints) and stiff finger grip (no bending at interphalangeal joints) as mentioned above.

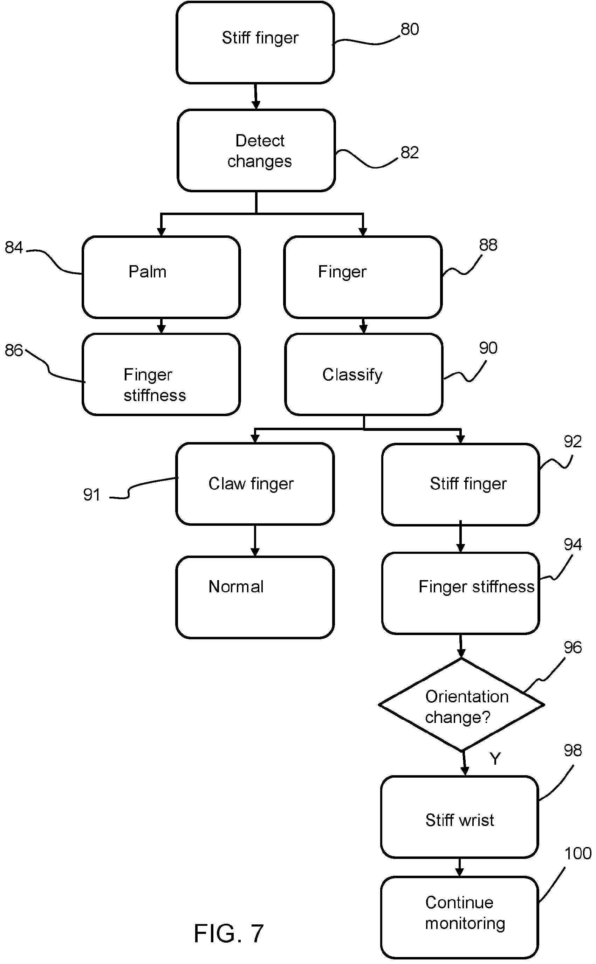

Fig. 7 shows an example of monitoring after a stiff finger grip is detected in step 80. Changes in grip pattern are monitored in step 82. If the next grip pattern is a palm grip as detected in step 84, this indicates a finger stiffness as assessed in step 86. If the next grip pattern is another finger grip as detected in step 88, it is again classified into a claw finger grip in step 91 or a stiff finger grip in step 92. A change from a stiff finger grip to a claw finger grip also indicates an absence of joint stiffness hence a normal response.

If the stiff finger grip is repeated, this confirms finger stiffness, as assessed in step 94. However, if the device is repositioned during release from the stiff finger grip, as assessed in step 96, this is a strong indicator of wrist problems as explained above, as assessed in step 98.

No repositioning the device during the release times indicates the wrist still has a good reach, but the stiff finger grip still indicates finger joint problems. The monitoring continues in step 100.

In addition to adapting the hand-held device for diagnosis of motoric problems it is also possible to modify the personal care device (e.g., powered toothbrush, or oral irrigator or shaver) for delivery of therapy. Specifically, such devices are held every morning for around two minutes during the health care routine. Heating and optionally cooling may be applied to deliver thermal therapy whilst the user brushes their teeth or performs other daily personal care routines.

Delivery of heating and/or cooling to an entire device requires relatively high power levels and is also rather slow in its response (as there is a considerable thermal mass which requires heating or cooling). Therefore, in a preferred example, delivery of the heating or cooling therapy is focused around the affected joints. By knowing the grip pattern, local areas where joints are located can be inferred.

In a simplest example, the complete handle is cooled or warmed using for example resistive heating elements or large Peltier elements or even small refrigeration units. However, such an approach is very power hungry. Heating or cooling is instead preferably only applied to the joints. For example, whilst brushing the teeth, the user can comfortably wrap all fingers (and indeed the thumb) around the toothbrush handle. Based on the known hand position when the handle is held in the intended manner, the heating and/or cooling can be focused to the position where the joints contact the handle.

As such, one example of a suitable heating/cooling element construction is a series of narrow heating or cooling elements spaced at the distance between the fingers. Thus, the heating elements are aligned with the intended paths followed by the fingers. In order to better align the fingers with the heating elements, a series of concentric ridges may be interspersed with the heating/cooling elements. These ridges guide the fingers mechanically to the intended positions. In this manner, a considerable saving in heating and cooling power can be realized. It may even be possible to heat or cool only a section of such a heating element at exactly the position of the joint. However, in some cases this could require sensing to identify the joint position. In particular, during brushing, the user rotates the brush around their grip, such that joints are positioned at different points around the circumference of the handle at different points in the cleaning routine. An element covering the full circumference is therefore a practical solution. However, the system response will also change when rotating and gripping different orientations so that rotation may also be detected based on this non-linear system response. A 3D accelerometer may also be used to track such movements.

In many cases, certain joints are more painful than others. This will be known prior to brushing (as joint pain only develops slowly). In such cases it is possible to focus the heating or cooling only to joints which have pain. This can be achieved by constructing the elements in an array of elements such as individually addressable heating and/or cooling strips.

Many different elements can advantageously be integrated into the handle but most suitable are designs that can be provided in a relatively thin form factor and wrapped around the curved handle. Examples are resistive heaters, IR LED strips, and IR LEDs connected to a light guide designed to emit light when a hand contact the lightguide.

Cooling elements may be in the form of Peltier elements.

A thermal therapy may involve alternating heating and cooling, and the particular therapy may depend on a type of arthritis (inflammatory vs. osteo arthritis).

The device may be configured for delivering a suitable therapy using a connected smartphone. The smartphone app may prepare the device for therapy based on the predicted times when the device is to be used. For example, such an app can determine when a person is awake so that the heating and cooling element can be prepared in advance of the use of the device, based on the average daily time between awakening and the personal care routine for a particular user) to provide a "warm" or "cool" brushing experience.

Alternatively, when the device is ready for the heating or cooling therapy, the app can give a notification to user that the device is ready and the individual can proceed with their personal care session.

In the example above, the processor is shown as part of the toothbrush. Of course, the signal processing may be performed remotely, and the personal care device may transmit data to the remote processing system to perform the analysis and return the results. The signal processing analysis may be performed in real time or using data stored during the personal care (or health care) routine.

The invention has been described with reference to a toothbrush, but the same concept may be applied to any hand-held device which is actuated such that it vibrates in use. The invention involves the detection of grip types based on the detectable interaction between the driven vibrations and the user's grip.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

Functions implemented by a processor may be implemented by a single processor or by multiple separate processing units which may together be considered to constitute a "processor". Such processing units may in some cases be remote from each other and communicate with each other in a wired or wireless manner (optional)

The mere fact that certain measures are recited in mutually different dependent claims does not indicate that a combination of these measures cannot be used to advantage.

If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

Any reference signs in the claims should not be construed as limiting the scope.

## Claims

1. A hand-held personal care device comprising:
a handle (14);
an actuator (16) for implementing a personal care function which generates a vibration;
a sensor arrangement (20) for detecting a signal which varies in dependence on the vibration of the personal care device;
a processing arrangement (22), configured to:
detect a user's grip pattern on the handle, in use of the personal care device, by sensing, using the sensor arrangement, a modulation of the vibrations generated by the actuator depending on the user's grip pattern.

2. The device of claim 1, wherein the processing arrangement is configured to classify the user's grip pattern as a palm grip or a finger grip or a combined palm and finger grip.

3. The device of claim 2, wherein the processing arrangement is configured to classify the finger grip as a stiff finger grip or a claw finger grip.

4. The device of any one of claims 1 to 3, wherein the processing arrangement comprises a neural network.

5. The device of claim 4, wherein the neural network is supplied with a spectrogram of the sensor signal for a continuous range of frequencies up to at least double the frequency of the vibrations generated by the actuator.

6. The device of any one of claims 1 to 5, wherein the processing arrangement is further configured to:
monitor changes in grip pattern over time; and
assess a presence and/or progression of a physical limitation of the user in generating a hand grip based on the grip pattern changes over time.

7. The device of claim 6, wherein the processing arrangement is configured to:
determine if the grip pattern changes from a first grip to a second grip with a grip pause between.

8. The device of claim 7, wherein the processing arrangement is configured to:
determine if the device orientation changes between first and second palm grips and provide an indication of wrist stiffness in response.

9. The device of claim 6, wherein the processing arrangement is configured to provide:
an indication of finger stiffness in response to a change from a stiff finger grip to a palm grip; or
an indication of finger stiffness in response to a change from a stiff finger grip to another stiff finger grip; or
an indication of an absence of joint stiffness in response to a change from a stiff finger grip to a claw finger grip; or
an indication of wrist stiffness in response to repositioning of the device during release between successive stiff finger grips.

10. The device of any one of claims 1 to 9, wherein the sensor arrangement comprises:
an accelerometer and/or a gyroscope; or
a current sensor for sensing an actuator drive signal.

11. The device of any one of claims 1 to 10, further comprising a therapy delivery system for delivering thermal therapy to the user.

12. The device of claim 11, wherein the therapy delivery system comprises a heating or cooling system for delivering heating or cooling targeted to the finger joints.

13. The device of any one of claims 1 to 12 comprising a powered toothbrush.

14. A method of detecting a user's grip pattern, comprising:
receiving a sensor signal which varies in dependence on a vibration of a hand-held personal care device which is actuated to implement a personal care function which involves driven vibrations; and
processing the sensor signal to detect a modulation of the vibrations generated by the actuator depending on the user's grip pattern and thereby determine the grip pattern.

15. A computer program comprising computer program code means which is adapted, when said program is run on a processor, to perform the method of claim 14.
